# EUROPEAN PATENT APPLICATION

(11) **EP 3 006 939 A1**
(43) Date of publication of application: **13.04.2016**
(21) Application number: 14187766.2
(22) Date of filing: 06.10.2014
(51) Int. Cl.: G01N 33/566, G01N 33/53

(54) **Histidine-rich Glycoprotein as a marker for hepatic Farnesoid X receptor activation**

(71) Applicant: Gilead Sciences, Inc., Foster City, CA 94404 (US)
(72) Inventor: Deuschle, Ulrich, 67363 Lustadt (DE)
(74) Representative: Grünecker Patent- und Rechtsanwälte PartG mbB

(57) **Abstract**

The invention relates to an in vitro method of detecting or monitoring the liver specific transcriptional activity or pharmacological activation of FXR in a human being or mammal, comprising quantifying the expression level of histidine-rich glycoprotein in the human being or mammal. The invention also relates to histidine-rich glycoprotein as a biomarker for monitoring FXR pharmacodynamic responsiveness of a human being or mammal to a FXR activity modulator. Furthermore, the invention relates to the use of histidine-rich glycoprotein for detecting or monitoring liver specific FXR activation in a human being or mammal.

SEQUENCE LISTING

<110>  Phenex Pharmaceuticals AG

<120>  Histidine-rich Glycoprotein as a marker for hepatic Farnesoid X
       receptor activation

<130>  EP96850FZ200pau

<140>  not yet assigned
<141>  herewith

<160>  15

<170>  PatentIn version 3.5

<210>  1
<211>  18
<212>  DNA
<213>  Artificial Sequence

<220>
<223>  Forward Primer

<400>  1
ttctgggatt gtaccgca                                                    18


<210>  2
<211>  16
<212>  DNA
<213>  Artificial Sequence

<220>
<223>  Reverse Primer

<400>  2
agcaaaccgc ttggga                                                      16


<210>  3
<211>  21
<212>  DNA
<213>  Artificial Sequence

<220>
<223>  Sequence Probe

<400>  3
cgtgcccgaa acgccgaata t                                                21


<210>  4
<211>  21
<212>  DNA
<213>  Artificial Sequence

<220>
<223>  Forward Primer

<400>  4
ggctaccttt tccaattgct g                                                21

<210>  5
<211>  21
<212>  DNA
<213>  Artificial Sequence

<220>
<223>  Reverse Primer

<400>  5
ggctcacagt cattccagta t                                                21


<210>  6
<211>  24
<212>  DNA
<213>  Artificial Sequence

<220>
<223>  Sequence Probe

<400>  6
accgaacagt ccgattcttg caca                                             24


<210>  7
<211>  20
<212>  DNA
<213>  Artificial Sequence

<220>
<223>  Forward Primer

<400>  7
ttagccccaa ggaatatgcc                                                  20


<210>  8
<211>  19
<212>  DNA
<213>  Artificial Sequence

<220>
<223>  Reverse Primer

<400>  8
gggttccagg acttcacac                                                   19


<210>  9
<211>  22
<212>  DNA
<213>  Artificial Sequence

<220>
<223>  Sequence Probe

<400>  9
tcctcttcaa ccccgatgtg cc                                               22


<210>  10
<211>  30

<212>  DNA
<213>  Artificial Sequence

<220>
<223>  Oligonucleotide

<400>  10
gcatgctcga ggcctcttcc catcccctca                                        30


<210>  11
<211>  33
<212>  DNA
<213>  Artificial Sequence

<220>
<223>  Oligonucleotide

<400>  11
gatcgaagct tcttgctatg atctgccact gca                                    33


<210>  12
<211>  30
<212>  DNA
<213>  Artificial Sequence

<220>
<223>  Primer

<400>  12
tgtgctcttc actgttccac ccactcatca                                        30


<210>  13
<211>  30
<212>  DNA
<213>  Artificial Sequence

<220>
<223>  Primer

<400>  13
tgatgagtgg gtggaacagt gaagagcaca                                        30


<210>  14
<211>  34
<212>  DNA
<213>  Artificial Sequence

<220>
<223>  Primer

<400>  14
ggttatttga agggaccctc aaggtgaaca ccct                                   34


<210>  15
<211>  34
<212>  DNA
<213>  Artificial Sequence

<220>
<223>  Primer

<400>  15
tgttcacctt gagggtccct tcaaataacc atca                                    34

## Description

The farnesoid X receptor (FXR) belongs to the nuclear receptor family of proteins, is expressed predominantly in tissues exposed to high levels of bile acids, binds bile acids as endogenous ligands and controls bile acid and lipid homeostasis (H. Higashiyama et al., Acta Histochem. 2008, 110, 86; A. L. Bookout et al., Cell 2006, 126, 789; B. Goodwin et al., Mol. Cell. 2000, 6, 517). In particular, activation of FXR by non-steroidal agonists results in strong reduction of total cholesterol and triglycerides in plasma and liver of wild-type but not FXR^{-/-} mice, leading to anti-atherosclerotic activity in an atherosclerosis model of human CETP transgenic LDLW^{-/-} mice (E. Hambruch et al., J. Pharmacol. Exp. Ther. 2012, 343, 556; M. J. Evans et al., Am. J. Physiol. Gastrointest. Liver Physiol. 2009, 296, G543). FXR also exerts tumor-protective functions in enterohepatic tissues as FXR^{-/-} mice develop hepatocellular carcinoma (HCC) at 12-15 months of age and show an increased prevalence for intestinal malignancies (F. Yang et al., Cancer Res. 2007, 67, 863; S. Modica et al., Cancer Res. 2008, 68, 9589). The expression of FXR is reduced in patients with non-alcoholic liver disease (Z.-X. Yang et al., Hepatol. Int. 2010, 4, 741) and experimental evidence gained mostly in animal models suggest that both steroidal and non-steroidal FXR agonists could be effective in the treatment of Non-Alcoholic Steatohepatitis ("NASH", L. Adorini et al., Drug Discovery Today 2012, 17, 988). Obeticholic Acid (6-Ethyl-Chenodeoxycholic acid, INT747), a semisynthetic steroidal FXR agonist, has been tested in clinical phase II studies in patients with type 2 diabetes and non-alcoholic steatohepatitis (S. Mudaliar et al., Gastroenterology 2013, 145, 574). In this phase II study, the insulin sensitivity in patients, as a primary read-out for treatment efficacy, was shown to be increased after the 6-week treatment period compared to baseline. As surrogate markers of FXR activation by Obeticholic acid, fibroblast growth factor 19, 7α-hydroxy-4-cholesten-3-one (a bile acid precursor) and endogenous bile acids were determined. Fibroblast growth factor 19 (FGF-19) is considered to be a direct transcriptional target of FXR primarily in the small intestine and after release into the portal blood stream travels to the liver to bind to fibroblast growth factor receptor 4 (FGFR4) present on hepatocytes. Activation of FGFR4 by FGF19 is thought to result in a reduction of expression of CYP7A1, the rate limiting enzyme for synthesis of the bile acid precursor 7α-hydroxy-4-cholesten-3-one ("C4"), leading to a reduction of plasma bile acids (P. Lefebvre et al., Physiol. Rev. 2009, 89, 147; L. Adorini et al., Drug Discov. Today 2012, 17, 988). The prominent role of intestinal specific activation of FXR on gene expression of key enzymes involved in bile acid synthesis has been demonstrated by using genetically modified mice (B. Kong et al., Hepatology 2012, 56, 1034). However, based on current knowledge it cannot be concluded if suppression of hepatic "C4" production is a readout for the action of intestine-derived FGF15/19 acting on hepatic FGF-Receptor 4 and subsequent downstream signaling or if liver intrinsic regulation of bile acid synthesis, in general, and Cyp7A1 mRNA levels, in particular, are dominant. There may also be species-specific differences between the impact of intestinal or hepatic FXR activation on bile acid production and C4-synthesis. For the treatment of liver diseases such as Non-alcoholic Fatty Liver Disease (NAFLD) or NASH it is widely assumed that liver specific FXR activation plays a pivotal role to counteract the disease-associated histopathology.

The mRNAs of KNG1 and FETUB have previously been shown to be directly up-regulated by steroidal and non-steroidal agonists of FXR in primary human hepatocytes (A. Zhao et al., J. Biol. Chem. 2003, 278, 28765; T. Murakami et al., Biochem. J. 2007, 407, 461). However, no data on the potential up-regulation and secretion of KNG1 and FETUB proteins in primary human hepatocyte cultures were presented. It was also not clear whether a potential up-regulation of either protein would be up-regulated in the serum or plasma of human subjects receiving an effective dose of a FXR agonist.

Thus, there is an urgent and stringent need for specific markers that could be used to directly and easily determine liver specific activation of FXR, ideally from plasma samples of patients in need of diagnosis or treatment to avoid error-prone and invasive liver biopsies.

### SUMMARY OF THE INVENTION

In this invention, the gene encoding histidine-rich glycoprotein (HRG, NM_000412.3, NP_000403.1, P04196) was identified as a direct transcriptional target gene of FXR in different human hepatoma cells and primary hepatocytes *in vitro.* Furthermore, we show that the HRG protein is also up-regulated as a secreted protein in the culture supernatant of the HepG2-FXR5 cells stably expressing human FXR, upon treatment with the non-steroidal FXR agonist PX20606 (E. Hambruch et al., J. Pharmacol. Exp. Ther. 2012, 343, 556). We show that HRG is specifically expressed in the liver in humans. Finally, in a placebo-controlled phase I safety study (ClinicalTrials.gov Identifier: NCT01998672), we identified HRG protein as significantly up-regulated in the plasma of healthy volunteers on day seven of daily oral administration of 0.5 mg/kg of Px-102 (an oral formulation of a GMP batch of PX20606) compared to the levels at beginning of the treatment. No significant change of plasma HRG levels was determined in plasma samples of healthy volunteers receiving daily oral administration of a vehicle suspension on day seven compared to the levels at the beginning of the treatment.

Histidine-rich glycoprotein (HRG) is a member of the Cystatin type 3 family together with Kininogen 1 (KNG1) and Kininogen 2 (only mice and rats, Kng2), Fetuin-beta (FETUB) and alpha-2-HS-glycoprotein (AHSG). HRG is composed of multiple structural domains conferring the function of an adaptor molecule binding to diverse ligands such as heparin, phospholipids, plasminogen, fibrinogen, IgG, complement C1q, heme and Zn²⁺, possibly explaining some of HRG's numerous proposed functions, namely the clearance of immune complexes, necrotic cells and pathogens and the modulation of angiogenesis, coagulation and fibrinolysis (C. Lee et al., Front. Biosci. 2009, 14, 2911, I. K. Poon et al., Blood 2011, 117, 2093).

### BRIEF DESCRIPTION OF THE DRAWINGS

**Figure 1****:** Huh7 cells, grown in quadruplicates in 12 well plates at a density of around 80%, were further incubated in growth medium supplemented with DMSO or 1 µM PX20606 for 24 h, as indicated. The relative fold induction of HRG and SHP mRNA's over *TBP* mRNA as an internal control were determined by RT-qPCR.

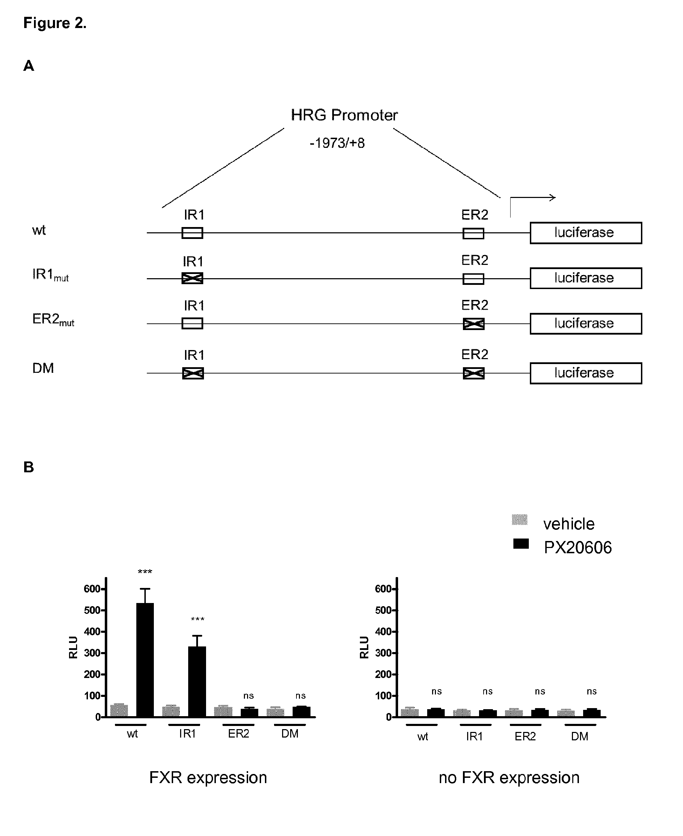

**Figure 2****:** Identification of functional FXR response elements in a human HRG promoter fragment. **A,** Schematic representation of the HRG promoter (position -1,973/+8 with respect to transcriptional start site). Candidate IR1 and ER2 sites are indicated as wt (open box) or a mutated sequences (crossed box). **B,** Luciferase reporter assays from Huh7 cells co-transfected with pTReX-Dest30-hFXRa2 (FXR expression), pCMV-RL and one of four pGL2-luciferase reporter plasmids containing the wild type HRG promoter fragment (wt) or mutated HRG promoter fragments carrying the single mutated (IR1) or (ER2) elements or the double mutated element (DM). The firefly luciferase activities normalized to Renilla luciferase activities given as relative light units (RLU) in presence of 1 µM PX20606 (black columns) versus DMSO as vehicle control (grey columns) of quadruplicate experiments are shown. Luciferase reporter assays from cells co-transfected with the parent vector pTreX-Dest30 (no FXR expression) are indicated.
**Figure 3****:** Upcyte^{®} hepatocytes were grown in triplicates for 24 hours in presence of DMSO or 1 µM PX20606 in DMSO. The relative fold induction of *HRG* and *SHP* mRNAs over *TBP* mRNA as internal control were determined by RT-qPCR.
**Figure 4****:** HepG2-FXR5 cells were grown in triplicates for 24 hours in presence of DMSO or 1 µM PX20606 in DMSO. The relative fold induction of *HRG* and *SHP* mRNAs over *TBP* mRNA as internal control were determined by RT-qPCR.
**Figure 5****:** Detection of HRG by Western Blot from the supernatants of HepG2-FXR5 cells. HRG was collected by Ni-NTA-Agarose from the supernatant of HepG2-FXR5 cells grown in medium with DMSO (D) or 1 µM PX20606 (PX) and detected by Western Blot.
**Figure 6****:** Distribution of human HRG mRNA in normal human tissues. A commercially available cDNA panel (normalized to GAPDH) from 48 different normal human tissues was tested by qPCR for relative abundance of HRG mRNA.
**Figure 7****:** HRG protein levels determined by ELISA from volunteers before (0 h) and after oral administration (6 h) of a single bolus of vehicle (placebo, n = 2) or 4.5 mg/kg of Px-102 (n = 6) were determined by ELISA.
**Figure 8****:** Plasma HRG protein levels in healthy volunteers are significantly increased after repeated dosing of Px-102 (PX20606) for 6 days. HRG plasma levels determined by ELISA from volunteers daily receiving orally either placebo (Vehicle, n = 9) or 0.5 mg/kg of Px-102 (n = 9). Plasma samples were derived from day 1 and day 7 right after dosing respectively. Plasma HRG levels were determined by ELISA in duplicates and represented as scatter plots with means and statistical significance determined using a non-paired Student's t-test.

### DETAILED DESCRIPTION OF THE INVENTION

The present invention provides an in vitro method of detecting or monitoring the liver specific transcriptional activity or pharmacological activation of FXR in a human being or mammal, comprising quantifying the expression level of histidine-rich glycoprotein in the human being or mammal.

The present invention provides that the gene coding for histidine-rich glycoprotein is a direct transcriptional target gene for FXR. In different human hepatoma cell lines and normal human hepatocytes, treatment with a steroidal or non-steroidal FXR modulator (preferably non-steroidal FXR agonist) leads to up-regulation of the HRG mRNA.

In a preferred embodiment the method comprises the quantification of the expression level of histidine-rich glycoprotein in a human being or mammal under treatment with a FXR activity modulator and the method further comprises quantifying the expression level of histidine-rich glycoprotein in an untreated host and comparing the expression levels. In a further preferred embodiment of the present invention the expression level is quantified in a human being or mammal before treatment with a FXR activity modulator and the method further comprises quantifying the expression level of histidine-rich glycoprotein in the same human being or mammal after single dose treatment, or after or during multiple dose treatment with a FXR activity modulator and comparing the expression levels.

In a preferred embodiment of the method according to the present invention the expression level is quantified by measuring the protein or mRNA level of histidine-rich glycoprotein in plasma or serum samples or liver biopsies.

The present invention also provides histidine-rich glycoprotein as a biomarker for monitoring FXR pharmacodynamic responsiveness of a human being or mammal to a FXR activity modulator thereby allowing for quantitative comparisons of the liver specific FXR potency of such FXR modulators.

The present invention further provides the use of histidine-rich glycoprotein for detecting or monitoring liver specific FXR transcriptional activity or pharmacological activation in a human being or mammal. In a preferred embodiment the histidine-rich glycoprotein is used by quantifying histidine-rich glycoprotein levels in plasma or serum samples of the human being or mammal.

In a preferred embodiment of the present invention the FXR activity modulator is a small drug-like molecule.

The present invention further provides FXR activity modulators, preferably FXR agonists, for use in the treatment, prevention or prophylaxis of diseases that are characterized by a reduction of histidine-rich glycoprotein expression. Such diseases are known to the skilled person, for example, solid cancers, liver failure, sepsis and preeclampsia.

The term "FXR activity modulator" as used herein refers to small molecules that bind to FXR and modulate its activity. Small molecule, drug-like FXR activity modulators are described in literature, e.g. by D. Merk et al. Future Med. Chem. 2012, 4, 1015. Examples are 6-ECDCA (Int-747), GW4064, FXR-450, Fexaramine, MFA-1 or the benzimidazole-based FXR ligands from Roche. Additional small molecule FXR activity modulators are described in WO 2000/037077, WO 2003/015771, WO 2004/048349, WO 2007/076260, WO 2007/092751, WO 2007/140174, WO 2007/140183, WO 2008/025539, WO 2008/025540, WO 2008/051942, WO 2008/157270, WO 2009/005998, WO 2009/012125, WO 2009/149795, WO 2011/020615, WO 2013/007387, WO 2013/087519, WO 2013/087520, WO 2013/087521.

The term "liver specific" means that exclusively the transcriptional activity or pharmacological activation of FXR in the liver of a human being or mammal is detected or monitored. The FXR transcriptional activity or pharmacological activation in other tissues is not encompassed. Detecting refers to a singular measurement of a relevant parameter, while monitoring refers to a longitudinal observation of a relevant parameter.

The term "quantifying the expression level" as used herein refers to the measurement of the absolute or relative amounts of mRNA or protein of histidine-rich glycoprotein. The relative amount of histidine-rich glycoprotein is determined relative to a reference sample. Methods for quantifying the expression level are known to the person skilled in the art and may be for example microarray analysis, quantitative PCR, quantitative mass spectrometry, gene reporter assays, or enzyme linked immunosorbent assay (ELISA). Microarray analysis may be carried out using a Sentrix-6 V3array from Illumina Inc. San Diego U.S.A.. Quantitative PCR may be carried out using a ABI 7900HT Sequence Detection System from Applied Biosystems Darmstadt Germany.

The term "transcriptional activity" refers to the direct regulative effect that FXR has on the rate of transcription of its cognate target genes, in particular histidine-rich glycoprotein. This effect is exerted by direct binding of FXR to DNA.

The term "pharmacological activation" refers to the activation of the transcriptional activity of FXR by direct binding to a pharmaceutical agent, in particular small drug-like molecules. FXR may be activated by binding to the FXR activity modulators according to the present invention. The term "pharmacodynamic responsiveness" refers to the degree of biochemical and physiological response to a pharmaceutical agent, in particular a FXR activity modulator according to the present invention.

The term "small drug-like molecule" refers to a small organic compound that has a molecular weight of less than 1000 Da.

### EXAMPLES

Commercially available reagents and kits referred to in the examples were used according to the manufacturer's instructions unless otherwise indicated. PX20606 (4-(2-(2-chloro-4-((5-cyclopropyl-3-(2,6-dichlorophenyl)isoxazol-4-yl)methoxy)phenyl)cyclopropyl)benzoic acid as disclosed in WO 2011/020615 and E. Hambruch et al., J. Pharmacol. Exp. Ther. 2012, 343, 556) was synthesized according to published synthesis protocols (WO 2011/020615) and checked for identity and purity by ¹H-NMR and LC-MS before being tested in FXR activity assays. If not otherwise stated, data are presented as the mean ± standard error of mean (± SEM) from at least three experimental measurements. Differences between experimental groups were evaluated for statistical significance using Student's t-test: *, p<0.05; **, p<0.01; ***, p<0.001 using the software Prism5 (GraphPad Software Inc., San Diego, USA). *p<0.05 was considered to be statistically significant.

### Example 1: Microarray experiment

HepaRG cells were obtained plated from Biopredic International (# HPR203-MW96, Rennes, France) and cultivated in 6-well plates using the media and protocol supplied by the manufacturer. Cells were cultivated in induction medium (Biopredic International) with 1 µM of PX20350 (E. Hambruch et al., J. Pharmacol. Exp. Ther. 2012, 343, 556) diluted from a 20 mM stock solution in DMSO or DMSO as a vehicle control for 24 hours. Total RNA was isolated following the instructions of the RNeasy 96 RNA isolation kit (Qiagen, Hilde, Germany) and 200 ng total RNA used for a genome wide mRNA array analysis done as a fee for service at the Genomics & Pro-teomics Core Facility of the German Research Facility using the human Sentrix-6 V3 (4213572043) array from Illumina and standard experimental kits and protocols supplied by the manufacturer (Illumina Inc. San Diego U.S.A.).

HRG mRNA was identified as a new gene upregulated (2-fold) by the FXR agonist compared to DMSO as a vehicle treatment. This upregulation was similar to that (2.7 fold) of the known direct FXR target gene NR0B2 (SHP, Small heterodimer partner). In comparison, TBP mRNA (TATA box binding protein) was not changed in relative abundance (Table 1).

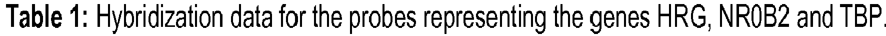

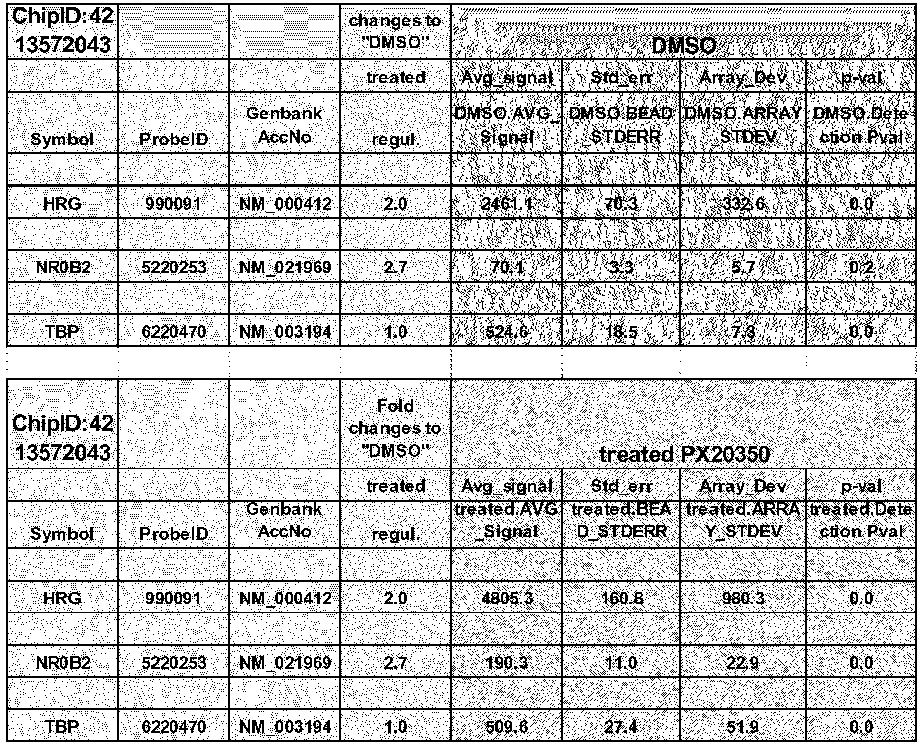

### Example 2: Induction of HRG and SHP mRNAs by PX20606 in Huh7 human hepatoma cells

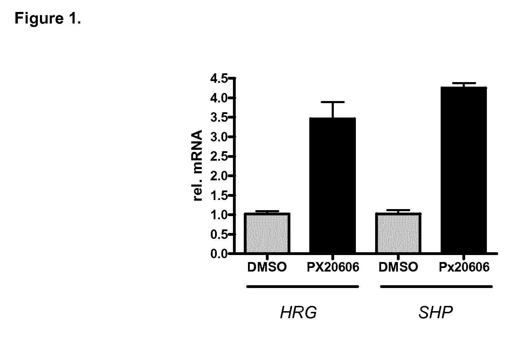

Huh7 (# JCRB0403) cells were obtained from the Health Science Research Resources Bank, Osaka, Japan. Cells were cultivated in MEM containing 8.6% FCS supplemented with 2 mM L-Alanyl-L-Glutamine (all purchased via Sigma-Aldrich, Munich, Germany) in presence of 1 µM of PX20606 or DMSO as a vehicle for 16 hours. Total RNA from cells grown in 96 well tissue plates (Huh7, HepaRG and 3D human liver microtisues) were isolated following the instructions of the RNeasy 96 RNA isolation kit (Qiagen, Hilde, Germany) and cDNA's were synthesized from 1/25^{th} of the isolated total RNA from one 96 well using Superscript II reverse Transcriptase (Life Technologies, Carlsbad, U.S.A.). Quantitative PCR was performed and analyzed using Absolute QPCR Rox Mix (Life Technologies, Carlsbad, U.S.A.) and a 384-format ABI 7900HT Sequence Detection System (Applied Biosystems, Darmstadt, Germany). Primer and probe sequences (purchased from IDT DNA Technologies, Leuven, Belgium) are listed in Table 2. Gene expression was expressed in arbitrary units and normalized relative to the housekeeping gene TATA box binding protein (TBP). The TBP normalized mRNA levels were plotted for HRG and SHP in Figure 1 (number of replicates n = 6).

**Table 2: Primer and probe sequences.**

| Gene | species | Refseq | Forward Primer | Reverse Primer | Sequence Probe |
|---|---|---|---|---|---|
| TBP | human | NM_003194 | TTCTGGGATTGTACCGCA (SEQ ID No. 1) | AGCAAACCGCTTGGGA (SEQ ID No. 2) | CGTGCCCGAAACGCCGAATAT (SEQ ID No. 3) |
| HRG | human | NM_000412 | GGCTACCTTTTCCAATTGCTG (SEQ ID No. 4) | GGCTCACAGTCATTCCAGTAT (SEQ ID No. 5) | ACCGAACAGTCCGATTCTTGCACA (SEQ ID No. 6) |
| SHP | human | NM_021969 | TTAGCCCCAAGGAATATGCC (SEQ ID No. 7) | GGGTTCCAGGACTTCACAC (SEQ ID No. 8) | TCCTCTTCAACCCCGATGTGCC (SEQ ID No. 9) |

HRG mRNA was shown to be upregulated in Huh7 hepatoma cells (around 3.5 fold) when compared to DMSO as a vehicle control. The known direct FXR target gene was also upregulated (around 4 fold) in this experiment (Fig. 1).

### Example 2: Identification of a FXR responsive DNA element in a fragment of the human HRG promoter by Luciferase reporter assays

A human HRG promoter fragment was cloned as a Xhol-Hindlll fragment (from position -1,973 till +8 with respect to the mRNA starting nucleotide at +1) using the oligonucleotides GCATGCTCGAGGCCTCTTCCCATCCCCTCA (SEQ ID No. 10) and GATCGAAGCTTCTTGCTATGATCTGCCACTGCA (SEQ ID No. 11) into the Xhol and Hindlll sites of pGL2basic (Promega, Madison, U.S.A.). The IR1 and ER2 sites were mutated using the primers of Table 3.

**Table 3: Primer sequences**

| |
|---|
| IR1mut5 TGTGCTCTTCACTGTTCCACCCACTCATCA (SEQ ID No. 12) |
| IR1mut3 TGATGAGTGGGTGGAACAGTGAAGAGCACA (SEQ ID No. 13) |
| ER2mut5 GGTTATTTGAAGGGACCCTCAAGGTGAACACCCT (SEQ ID No. 14) |
| ER2mut3 TGTTCACCTTGAGGGTCCCTTCAAATAACCATCA (SEQ ID No. 15) |

The reporter plasmids pGL2-FXR-RE (FXR-RE), pGL2-P_{HRG} (wt), pGL2-P_{HRG}-IR1mut (IR1mut) and pGL2-P_{HRG}-ER2mut (ER2mut) and pGL2-P_{HRG}-IR1/ER2 (DM) (each 50 ng/well), were co-transfected with pTRex-Dest30-hFXR (20 ng/well) and pCMV-RL (5 ng/well, constitutively expressing the *Renilla* luciferase under control of a CMV promotor as an internal control) into Huh7 cells using lipofection with Polyethylenimine and cultivated in presence or absence of 1 µM of the FXR agonist PX20606 or DMSO as a vehicle control for 16 h followed by cell lysis and determination of luciferase activities as described in U. Deuschle et al., PLoS One 2012, 7, e43044.

The Promoter fragment spanning from position -1973 to +8 (+1 is the transcriptional start of HRG mRNA) of the human HRG gene in pGL2-P_{HRG} (wt), shows no activity in transiently transfected Huh7 cells (Fig. 2B no FXR expression). When FXR is exogenously expressed, the promoter is active in presence of the FXR agonist PX20606 but not in absence (DMSO) (Fig. 2B FXR expression). Mutation of the IR-1 type putative FXR binding site does reduce the activity of the corresponding promoter, while mutation of the ER-2 type FXR binding sequence results in loss of FXR/FXR agonist responsitivity. These results show that the HRG promoter contains sequence elements that mediate FXR/FXR agonist inducibility to the HRG gene in Huh7 cells exogenously expressing human FXR. These sequences do possibly contribute to the inducibility of the human HRG gene transcription by FXR agonists in the human liver.

### Example 3: Induction of HRG transcription by FXR agonist PX20606 in human primary Upcyte^{®} Hepatocytes

Upcyte^{®} hepatocytes (Cat# UH0-000-KA00 donor 10-03) derived from primary hepatocytes of a female donor were cultivated in 96 well plates in high performance medium (Medicyte, Heidel-berg, Germany). Isolation of total RNA followed by quantitative qPCR was done as described in Example 2.

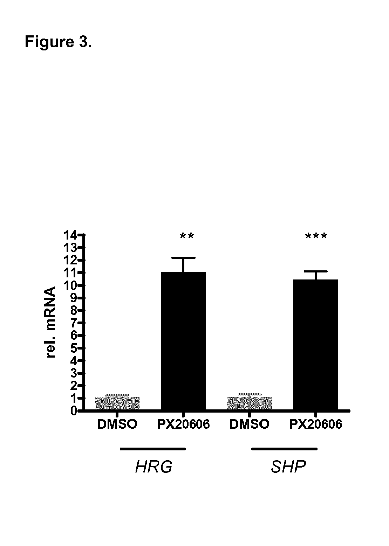

HRG mRNA was shown to be upregulated in Upcyte® hepatocytes (around 11 fold) when compared to DMSO as a vehicle control. The known direct FXR target gene was also upregulated (around 10 fold) in this experiment (Fig. 3).

### Example 4: Induction of HRG transcription by FXR agonist PX20606 in in HepG2-FXR5 cells

HepG2-FXR5 cells (described in U. Deuschle et al., PLoS One. 2012, 7, e43044) were cultivated in 96 well plates in RPMI medium supplemented with 8.6% FCS and supplemented with 2 mM L-Alanyl-L-Glutamine (all purchased via Sigma-Aldrich, Munich, Germany). Isolation of total RNA followed by quantitative qPCR was done as described in Example 2.

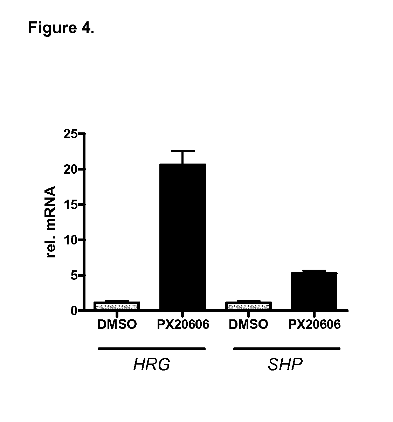

HRG mRNA was shown to be upregulated in HepG2-FXR5 hepatocytes (around 20 fold) when compared to DMSO as a vehicle control. The known direct FXR target gene was also upregulated (around 6 fold) in this experiment (Fig. 4).

### Example 5: Detection of secreted HRG in the supernatant of HepG2-FXR5 cells

HepG2-FXR5 cells (described in U. Deuschle et al., PLoS One. 2012, 7, e43044) were cultivated in T75 flasks in RPMI medium supplemented with 0,2% BSA, 2 mM L-Alanyl-L-Glutamine (Sigma-Aldrich, Munich, Germany) and 1x Insulin-Transferrin-Selenium (#51500-056, Life Technolgies, Carlsbad, USA). Each 10 ml supernatant from cells grown in presence of 1 µM PX20606 or DMSO as vehicle for 48 hours were mixed with 100 µL Ni-NTA agarose slurry (#1018240, Qiagen, Hilden, Germany) for 1 h, the slurry was collected by centrifugation and after 3 washes with 10 mL 1xPBS resuspended in 100 µL SDS sample buffer. Western blot was performed (as described in U. Deuschle et al., PLoS One 2012, 7, e43044) and HRG detected using polyclonal mouse-anti human HRG antibodies (ab67807, Abcam, Cambridge, UK).

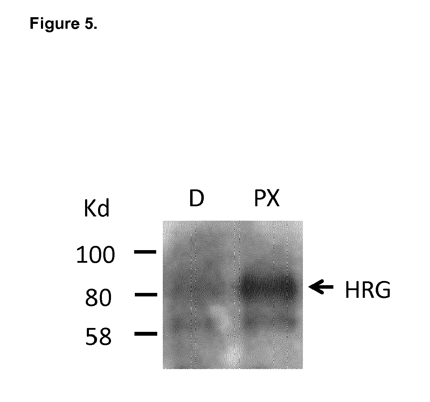

The secreted HRG was detected in the Western Blot as a band running at a position corresponding to around 85 kDa (Fig. 5). The amount of secreted HRG was increased when cells were cultivated in presence of 1 µM PX20606 for 48 hours.

### Example 6: Distribution of HRG mRNA in 48 normal human tissues

The distribution of HRG mRNA was done by qPCR using the HRG and TBP assays listed in Table 2 and a commercially available cDNA panel derived from 48 normal human tissues (#HMRT102, Origene, Rockville, USA).

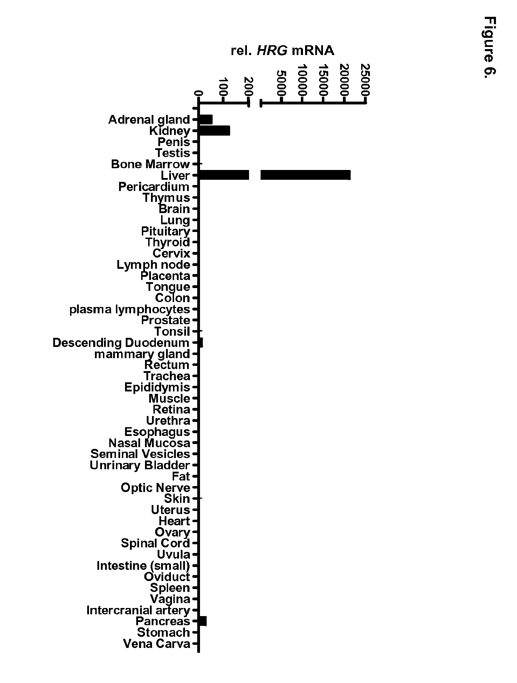

The relative (TBP corrected) HRG mRNA level is depicted in Figure 6, which shows that HRG is predominantly expressed in human liver.

### Example 7: HRG plasma levels in healthy volunteers - single bolus of FXR agonist

In one arm of a placebo controlled Single Ascending Dose study (ClinicalTrials.gov Identifier: NCT01998659), healthy male volunteers received a bolus of vehicle drinking solution (placebo, n = 2) or 4.5 mg/kg FXR agonist PX20606 in vehicle drinking solution (Px-102, n = 6). HRG protein levels in plasma drawn before 0 h and after 6 h of administration were determined by ELISA (# SEK10836, Sino Biological Inc., Bejing, P.R. China).

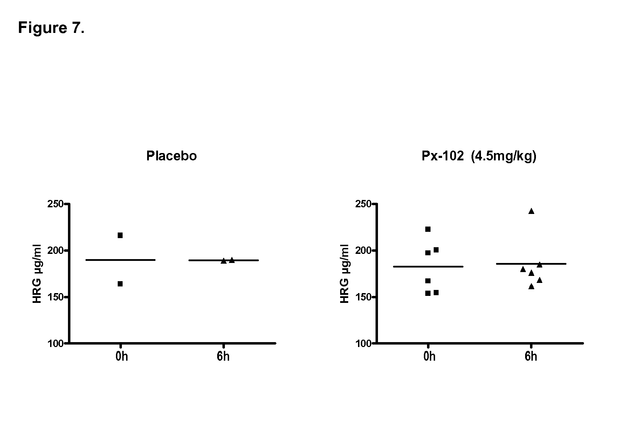

Plasma HRG levels did not change significantly during the first 6 hours after administration of vehicle or Px-102 (Fig. 7).

### Example 8: HRG plasma levels in healthy volunteers - multiple administration

In a placebo controlled Multiple Ascending Dose Study (ClinicalTrials.gov Identifier: NCT01998672), healthy male volunteers received either placebo (vehicle drinking solution, n = 9) or 0.5 mg/kg PX20606 in vehicle drinking solution (Px-102, n = 9) daily for consecutive 7 days. HRG plasma protein levels were determined by ELISA (# SEK10836, Sino Biological Inc., Bejing, P.R. China) from blood drawn on day 1 and day 7 right after administration or the respective drinking solution.

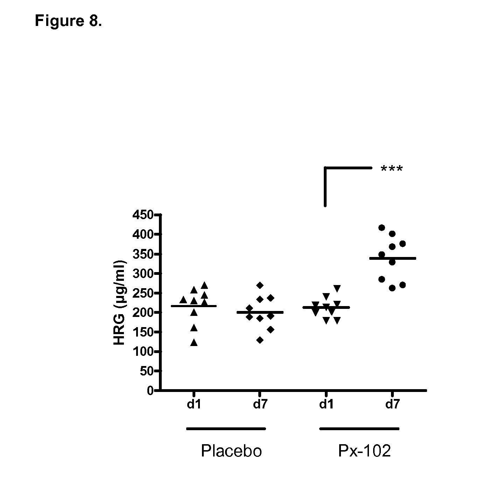

While no apparent change was detected for plasma HRG levels from volunteers receiving placebo, a significant increase of plasma HRG levels was determined with volunteers receiving the FXR agonist (Px-102) daily for 7 consecutive days (Fig. 8).

## Claims

1. An in vitro method of detecting or monitoring the liver specific transcriptional activity or pharmacological activation of FXR in a human being or mammal, comprising quantifying the expression level of histidine-rich glycoprotein in the human being or mammal.

2. The method of claim 1, wherein the expression level is quantified in a human being or mammal under treatment with a FXR activity modulator, further comprising quantifying the expression level of histidine-rich glycoprotein in an untreated human being or mammal and comparing the expression levels.

3. The method of claim 1, wherein the expression level is quantified in a human being or mammal before treatment with a FXR activity modulator, further comprising quantifying the expression level of histidine-rich glycoprotein in the same human being or mammal after single dose treatment, or after or during multiple dose treatment with a FXR activity modulator and comparing the expression levels.

4. The method of any of claims 1 to 3, wherein the expression level of histidine-rich glycoprotein is quantified by measuring the protein or mRNA level of histidine-rich glycoprotein in plasma or serum samples or liver biopsies.

5. Histidine-rich glycoprotein as a biomarker for monitoring FXR pharmacodynamic responsiveness of a human being or mammal to a FXR activity modulator thereby allowing for quantitative comparisons of the liver specific FXR potency of such FXR modulators.

6. Use of histidine-rich glycoprotein for determining liver specific FXR transcriptional activity or pharmacological activation in a human being or mammal.

7. The use of claim 6, wherein histidine-rich glycoprotein levels are quantified in plasma or serum samples of the human being or mammal.

8. The method or use of any of claims 1 to 7, wherein the FXR activity modulator is a small drug-like molecule.

9. An FXR activity modulator for use in the treatment of a disease wherein histidine-rich glycoprotein expression is reduced.
